# EUROPEAN PATENT APPLICATION

(11) **EP 4 353 707 A1**
(43) Date of publication of application: **17.04.2024**
(21) Application number: 22819201.9
(22) Date of filing: 14.04.2022
(51) Int. Cl.: C07C 51/265, C07C 63/06, C07C 63/331, C07C 45/36, C07C 49/67, C07C 49/786, C07C 29/50, C07C 31/04, C07C 51/215, C07C 53/02, C07D 231/12, C07C 27/12, C07B 33/00

(54) **METHOD FOR LIGHT-PROMOTED OXIDATION OF COMPOUND CONTAINING SATURATED CARBON-HYDROGEN BOND**

(30) Priority: 09.06.2021 CN 202110642049; 15.02.2022 CN 202210137499
(71) Applicant: Fudan University, Shanghai 200433 (CN)
(72) Inventor: SHI, Zhangjie, Shanghai 200438 (CN); MA, Shengming, Shanghai 200433 (CN); LIU, Feng, Shanghai 200438 (CN); YU, Hao, Shanghai 200433 (CN); WANG, Changcheng, Shanghai 200438 (CN); LIU, Qi, Shanghai 200433 (CN); ZHANG, Zhen, Shanghai 200438 (CN)
(74) Representative: Pfenning, Meinig & Partner mbB
(86) International application number: PCT/CN2022/086889
(87) International publication number: WO 2022/257598

(57) **Abstract**

The present invention belongs to the technical field of organic intermediate synthesis, and provides a method for light-promoted oxidation of a compound containing a saturated carbon-hydrogen bond, comprising: mixing a compound containing a saturated carbon-hydrogen bond with a catalyst, and oxidizing the compound containing a saturated carbon-hydrogen bond in an oxygen or air atmosphere at a temperature of 20°C to 100°C under light irradiation to generate an oxidation product. The present method is a method for the light-promoted direct oxidation of a compound containing a saturated carbon-hydrogen bond, which only requires a relatively low temperature to be carried out, has good compatibility with functional groups, short reaction time, high reaction efficiency, low reaction costs, high added value, simple operation and good safety, and is a mild, green and environmentally friendly oxidation method.

## Description

The present application claims the priorities of Chinese Patent Application No. 202110642049.7, filed before the CNIPA on June 09, 2021, titled "METHOD FOR LIGHT-PROMOTED OXIDATION OF COMPOUND CONTAINING SATURATED CARBON-HYDROGEN BOND", and Chinese Patent Application No. 202210137499.5, filed before the CNIPA on February 15, 2022, titled "METHOD FOR LIGHT-PROMOTED OXIDATION OF COMPOUND CONTAINING SATURATED CARBON-HYDROGEN BOND", which are incorporated herein by reference in their entirety.

### TECHNICAL FIELD

The present application belongs to the technical field of the synthesis of an organic intermediate, and specifically relates to a method for the light-promoted oxidation of a compound containing a saturated carbon-hydrogen bond.

### BACKGROUND

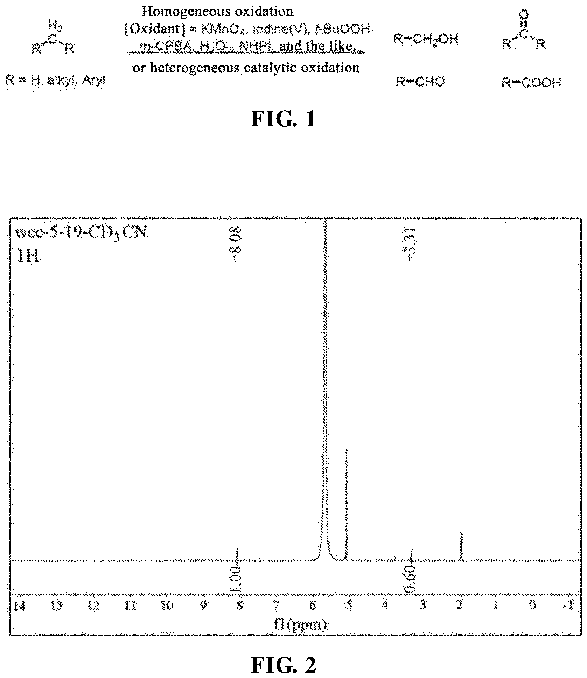

Oxygen-containing organic compounds such as alcohols, peroxy alcohols, aldehydes, ketones, carboxylic acids and peroxy acids have important applications in daily life and in the basic chemical industry, among which terephthalic acid is the raw material of polyester, with an annual output of hundreds of millions of tons; salicylic acid is widely used in personal health care and food preservation; and peroxyacetic acid is an eco-bactericidal agent and a bleaching agent for paper and wood. Oxidation reaction is a very important class of reactions in the chemical industry, and the majority of oxygen-containing compounds are prepared by oxidation reactions. As shown in FIG. 1, traditional oxidation generally adopts homogeneous oxidation or heterogeneous catalytic oxidation, and, for example, oxidant for homogeneous oxidation includes metallic oxidant (potassium permanganate (KMnO₄)), transition metal, diiodine pentaoxide (iodine V), peroxides (t-Butyl Hydroperoxide (t-BuOOH), meta-chloroperoxybenzoic acid (m-CPBA), hydrogen peroxide (H₂O₂)), and N-hydroxyphthalimide (NHPI). The above catalysts are high-consuming or expensive, restricting the development and application of oxidation reaction.

Under the guidance of sustainable development, it is imperative to develop new energy-saving and environmentally friendly synthesis methods. How to oxidize alkane compounds efficiently under mild and green conditions using inexpensive catalysts is a goal that has been pursued in the art of chemical synthesis. Organic oxidation reactions using oxygen as an oxidant have become a popular research direction due to their higher atom economy, low cost, and relatively small environmental damage, which meets the growing environmental requirements. In recent years, chemical reactions promoted by organometallic catalysts or organic dyes with photosensitive characteristics have been rapidly developed. Most of the currently developed light-promoted oxidation reactions require the use of expensive metallic catalysts such as ruthenium, platinum, iridium, and the like, or organic photosensitizers with complex structures such as methyl acridine, anthraquinone derivatives, and the like. The use of these two types of catalysts has certain shortcomings in terms of economy and environmental protection, which may increase the cost of the reaction and the pollution to the environment.

### SUMMARY

The present application was carried out to solve the above problems, and aims to provide an environmentally friendly method for light-promoted oxidation of a compound containing a saturated carbon-hydrogen bond without metal participation. The specific technical solution is as follows.

The present application provides a method for light-promoted oxidation of a compound containing a saturated carbon-hydrogen bond, comprising: mixing the compound containing the saturated carbon-hydrogen bond with a catalyst, and subjecting the compound containing the saturated carbon-hydrogen bond to an oxidation reaction in an oxygen or air atmosphere at a temperature of 20°C to 100°C under light irradiation to generate an oxidation product.

In the present application, the method for oxidation of a compound containing a saturated carbon-hydrogen bond refers to a method for oxidizing a carbon-hydrogen bond on a carbon atom that does not have an unsaturated bond.

The method for light-promoted oxidation of the compound containing the saturated carbon-hydrogen bond provided in the present application may also be characterized in that: the catalyst is any one of tert-butyl hypochlorite, hydrochloric acid, or hydrobromic acid; or the catalyst is a mixture capable of in-situ generating hydrochloric acid or hydrobromic acid, wherein the mixture is a mixture of chloride and acetic acid or of bromide and acetic acid.

The method for light-promoted oxidation of the compound containing the saturated carbon-hydrogen bond provided by the present application may also be characterized in that: the compound containing the saturated carbon-hydrogen bond is C1-C8 alkane.

The method for light-promoted oxidation of the compound containing the saturated carbon-hydrogen bond provided by the present application may also be characterized in that: the compound containing the saturated carbon-hydrogen bond is C1-C8 alkane substituted with phenyl, biphenyl or substituted phenyl, wherein a substituent of the substituted phenyl is any one selected from the group consisting of halogen, nitro, cyano, hydroxyl, amino, carboxyl, C2-C8 carboxylic ester, and 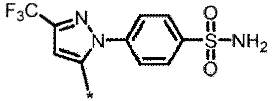 group.

The method for light-promoted oxidation of the compound containing the saturated carbon-hydrogen bond provided by the present application may also be characterized in that: the catalyst is an oxyacid, wherein the oxyacid is any one of p-toluenesulfonic acid, sulfuric acid or nitric acid.

The method for light-promoted oxidation of the compound containing the saturated carbon-hydrogen bond provided by the present application may also be characterized in that: the light has a wavelength of 300 nm to 800 nm.

The method for light-promoted oxidation of the compound containing the saturated carbon-hydrogen bond provided by the present application may also be characterized in that: the oxidation product is at least one of alcohol, peroxyalcohol, aldehyde, ketone, carboxylic acid or peroxyacid.

The method for light-promoted oxidation of the compound containing the saturated carbon-hydrogen bond provided by the present application may also be characterized in that: the oxygen or air atmosphere in a reaction system has a pressure of 1 atm to 100 atm.

The method for light-promoted oxidation of the compound containing the saturated carbon-hydrogen bond provided by the present application may also be characterized in that: a molar ratio of the compound containing the saturated carbon-hydrogen bond to the catalyst is 1:0.0001 to 1: 1.

The method for light-promoted oxidation of the compound containing the saturated carbon-hydrogen bond provided by the present application may also be characterized in that: a solvent for the oxidation reaction is any one of acetonitrile, tetrahydrofuran, dichloromethane, dichloroethane, trichloromethane, dimethyl sulfoxide, dibromoethane or N,N-dimethylformamide.

The present application provides a method for light-promoted oxidation of a compound containing a saturated carbon-hydrogen bond, wherein the compound containing the saturated carbon-hydrogen bond undergoes an oxidation reaction with oxygen in the presence of catalyst under a condition of light irradiation to synthesize a corresponding oxidation product, such as at least one of alcohol, peroxyalcohol, aldehyde, ketone, carboxylic acid, or peroxyacid. The method is a light-promoted direct oxidation method of a compound containing a saturated carbon-hydrogen bond, which only needs to be carried out at a low temperature (20°C to100°C), has the advantages of good compatibility of functional groups, short reaction time, high reaction efficiency, low reaction cost, high added value, simple operation, and good safety, and is a mild, green, and environmentally friendly oxidation method.

Of course, all of the advantages described above do not have to be achieved at the same time when any of the products or methods of the present application is implemented.

### BRIEF DESCRIPTION OF THE DRAWINGS

FIG. 1 is a schematic diagram of a traditional oxidation method;
FIG. 2 is a 1HNMR spectrum of the product in Example 15 of the present application.

### DETAILED DESCRIPTION

In order to make the technical means, inventive features, purposes and effects achieved by the present application easy to understand, the present application is specifically described in detail below in combination with embodiments and drawings, but the scope of protection of the present application is not limited to the specific embodiments. Other embodiments obtained by those skilled in the art according to the embodiments of the present application are also within the scope of protection of the present application.

The present application provides a method for light-promoted oxidation of a compound containing a saturated carbon-hydrogen bond, comprising: mixing the compound containing the saturated carbon-hydrogen bond with a catalyst, and subjecting the compound containing the saturated carbon-hydrogen bond to an oxidation reaction in an oxygen or air atmosphere at a temperature of 20°C to 100°C under light irradiation to generate an oxidation product.

In the present application, the method for oxidation of a compound containing a saturated carbon-hydrogen bond refers to a method for oxidizing a carbon-hydrogen bond on a carbon atom that does not have an unsaturated bond.

In some embodiments of the present application, the catalyst is any one of tert-butyl hypochlorite, hydrochloric acid, or hydrobromic acid; or the catalyst is a mixture capable of in-situ generating hydrochloric acid or hydrobromic acid, wherein the mixture is a mixture of chloride and acetic acid or of bromide and acetic acid. Among them, a molar ratio of chloride to acetic acid or of bromide to acetic acid is 1:1 to 1:10. Exemplarily, a mixture liquid of ammonium chloride and acetic acid at a molar ratio of 1:1 may be used as the catalyst.

In some embodiments of the present application, the compound containing the saturated carbon-hydrogen bond is C1-C8 alkane. In the present application, the C1-C8 alkane may be a liner alkane, a branched alkane, or a cycloalkane, including but not limited to methane, ethane, propane, butane, isobutane, pentane, isopentane, hexane, isohexane, heptane, isoheptane, octane, isooctane, cyclopropane, cyclobutane, cyclopentane, cyclohexane, cycloheptane, or cyclooctane.

In some embodiments of the present application, the compound containing the saturated carbon-hydrogen bond is C1-C8 alkane substituted with phenyl, biphenyl or substituted phenyl, wherein a substituent of the substituted phenyl is any one selected from the group consisting of halogen, nitro, cyano, hydroxyl, amino, carboxyl, C2-C8 carboxylic ester, and 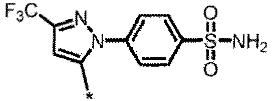 group, the halogen substituent is selected from the group consisting of fluorine, chlorine, bromine and iodine, and the substitution position of the above substituted phenyl may be ortho-position, meta-position or para-position.

In some embodiments of the present application, the catalyst is an oxyacid, wherein the oxyacid is any one of p-toluenesulfonic acid, sulfuric acid or nitric acid.

In some embodiments of the present application, the light has a wavelength of 300 nm to 800 nm.

In some embodiments of the present application, the oxidation product is at least one of alcohol, peroxyalcohol, aldehyde, ketone, carboxylic acid peroxyacid.

In some embodiments of the present application, the oxygen or air atmosphere in a reaction system has a pressure of 1 atm to 100 atm.

In some embodiments of the present application, a molar ratio of the compound containing the saturated carbon-hydrogen bond to the catalyst is 1:0.0001 to 1:1.

In some embodiments of the present application, a solvent for the oxidation reaction is any one of acetonitrile, tetrahydrofuran, dichloromethane, dichloroethane, trichloromethane, dimethyl sulfoxide, dibromoethane or N,N-dimethylformamide. In the present application, the reaction vessel used needs to enable the oxidation reaction promoted by light in a certain atmosphere, wherein the reaction vessel used includes, but is not limited to, a photoreactor or a quartz tube.

In the present application, there is no special restriction on the type of light source used, as long as the purpose of the present application can be achieved. In the present application, the power of the light source ranges from 1 W to 100 W. Exemplarily, a 40 W light-emitting diode (LED) lamp is used as the light source.

In the present application, the method of separating the target product after the light-promoted reaction is a separation method commonly used in art, such as filtration, vacuum distillation, extraction, and the like. The present application does not impose any limitation on the separation method, as long as the purpose of the present application can be achieved.

In the present application, the term "yield" refers to the molar percentage of the actual yield and the theoretical yield of a product; in the present application, the equation for calculating the yield is: yield of target product = actual number of moles of target product/theoretical number of moles of target product×100%.

Hereinafter, Examples and Comparative Examples are given to illustrate the embodiments of the present application more specifically. Various tests and evaluations were carried out according to the method described below. In addition, "portions" and "%" are used in the basis on weight unless otherwise specified.

### Test Methods and Equipment

A nuclear magnetic resonance spectrometer was used to determine the 1HNMR spectrum of the target product, to quantitatively determine the yield of the target product, and to identify the molecular structure of the target product.

### Example 1

### Preparation of benzoic acid:

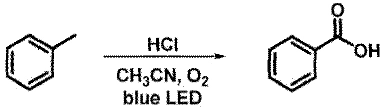

Toluene (92 mg, 1 mmol), hydrochloric acid (200 µL, 1 M), and acetonitrile (CH₃CN, 2 mL) were added in turn into a 25 mL quartz tube for mixing to obtain a mixture solution. The resulting mixture solution was irradiated with a blue LED lamp (40 W) with a wavelength of 380 nm to 550 nm in oxygen atmosphere (1 atm) for reacting for 12 h at room temperature. After the reaction was completed, the solvent was removed under reduced pressure to obtain 103 mg of the corresponding benzoic acid at a yield of 84%.

The 1HNMR spectrum of the product was:
¹H NMR (400 MHz, DMSO) *δ* 12.98 (s, 1H), 7.95 (d, *J* = 8.0 Hz, 2H), 7.63 (t, *J* = 7.2 Hz, 1H), 7.50 (t, *J* = 7.6 Hz, 2H). ¹³C NMR (100 MHz, DMSO) *δ* 167.4, 132.9, 130.8, 129.3, 128.6.

The reaction product was demonstrated to be the target product benzoic acid.

### Example 2

### Preparation of benzoic acid:

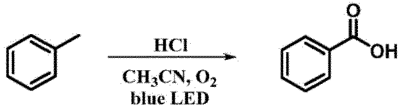

Example 2 differed from Example 1 in that the reaction temperature was adjusted to 60°C as follows.

Toluene (92 mg, 1 mmol), hydrochloric acid (200 µL, 1 M), and acetonitrile (2 mL) were added in turn into a 25 mL quartz tube for mixing to obtain a mixture solution. The resulting mixture solution was irradiated with a blue LED lamp (40 W) with a wavelength of 380 nm to 550 nm in oxygen atmosphere (1 atm) for reacting for 12 h at 60°C. After the reaction was completed, the solvent was removed under reduced pressure to obtain 109 mg of the corresponding benzoic acid at a yield of 89%.

### Example 3

### Preparation of benzoic acid:

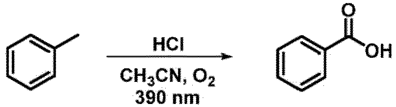

Example 3 differed from Example 1 in that the light source was adjusted to one with a wavelength of 390 nm as follows.

Toluene (92 mg, 1 mmol), hydrochloric acid (200 µL, 1 M), and acetonitrile (2 mL) were added in turn into a 25 mL quartz tube for mixing to obtain a mixture solution. The resulting mixture solution was irradiated with a LED lamp (40 W) with a wavelength of 390 nm in oxygen atmosphere (1 atm) for reacting for 12 h at room temperature. After the reaction was completed, the solvent was removed under reduced pressure to obtain 112 mg of the corresponding benzoic acid at a yield of 92%.

### Example 4

### Preparation of benzoic acid:

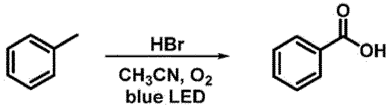

Example 4 differed from Example 1 in that hydrobromic acid was used as catalyst as follows.

Toluene (92 mg, 1 mmol), hydrobromic acid (200 µL, 1 M), and acetonitrile (2 mL) were added in turn into a 25 mL quartz tube for mixing to obtain a mixture solution. The resulting mixture solution was irradiated with a blue LED lamp (40 W) with a wavelength of 380 nm to 550 nm in oxygen atmosphere (1 atm) for reacting for 12 h at room temperature. The solvent was removed under reduced pressure to obtain 76 mg of the corresponding benzoic acid at a yield of 62%.

### Example 5

### Preparation of benzoic acid:

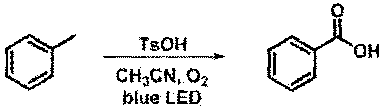

Example 5 differed from Example 1 in that p-toluenesulfonic acid was used as catalyst as follows.

Toluene (92 mg, 1 mmol), p-toluenesulfonic acid (TsOH, 200 µL, 1 M), and acetonitrile (2 mL) were added in turn into a 25 mL quartz tube for mixing to obtain a mixture solution. The resulting mixture solution was irradiated with a blue LED lamp (40 W) with a wavelength of 380 nm to 550 nm in oxygen atmosphere (1 atm) for reacting for 12 h at room temperature. The solvent was removed under reduced pressure to obtain 70 mg of the corresponding benzoic acid at a yield of 57%.

### Example 6

### Preparation of benzoic acid:

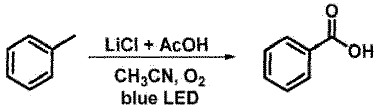

Example 6 differed from Example 1 in that a mixture liquid of lithium chloride and acetic acid was used as catalyst as follows.

Toluene (92 mg, 1 mmol), a mixture liquid of lithium chloride and acetic acid (at a molar ratio of LiCl: AcOH of 1:1, 200 µL, 1 M), and acetonitrile (2 mL) were added in turn into a 25 mL quartz tube for mixing. The mixture was irradiated with a blue LED lamp (40 W) with a wavelength of 380 nm to 550 nm in oxygen atmosphere (1 atm) for reacting for 12 h at room temperature. The solvent was removed under reduced pressure to obtain 92 mg of the corresponding benzoic acid at a yield of 75%.

### Example 7

### Preparation of benzoic acid:

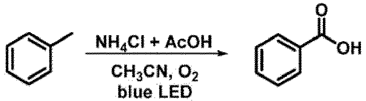

Example 7 differed from Example 1 in that a mixture liquid of ammonium chloride and acetic acid was used as catalyst as follows.

Toluene (92 mg, 1 mmol), a mixture liquid of ammonium chloride and acetic acid (at a molar ratio of NH₄Cl: AcOH of 1:1, 200 µL, 1 M), and acetonitrile (2 mL) were added in turn into a 25 mL quartz tube for mixing. The mixture was irradiated with a blue LED lamp (40 W) with a wavelength of 380 nm to 550 nm in oxygen atmosphere (1 atm) for reacting for 12 h at room temperature. The solvent was removed under reduced pressure to obtain 98 mg of the corresponding benzoic acid at a yield of 80%.

### Example 8

### Preparation of benzoic acid:

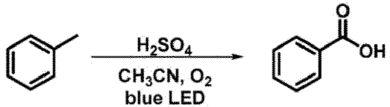

Example 8 differed from Example 1 in that sulfuric acid was used as catalyst as follows.

Toluene (92 mg, 1 mmol), sulfuric acid (200 µL, 1 M), and acetonitrile (2 mL) were added sequentially into a 25 mL quartz tube for mixing to obtain a mixture solution. The resulting mixture solution was irradiated with a blue LED lamp (40 W) with a wavelength of 380 nm to 550 nm in oxygen atmosphere (1 atm) for reacting for 12 h at room temperature. The solvent was removed under reduced pressure to obtain 105 mg of the corresponding benzoic acid at a yield of 86%.

### Example 9

### Preparation of benzoic acid:

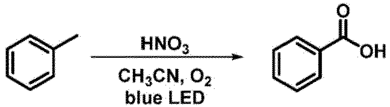

Example 9 differed from Example 1 in that nitric acid was used as catalyst as follows.

Toluene (92 mg, 1 mmol), nitric acid (200 µL, 1 M), and acetonitrile (2 mL) were added in turn into a 25 mL quartz tube for mixing to obtain a mixture solution. The resulting mixture solution was irradiated with a blue LED lamp (40 W) with a wavelength of 380 nm to 550 nm in oxygen atmosphere (1 atm) for reacting for 12 h at room temperature. The solvent was removed under reduced pressure to obtain 102 mg of the corresponding benzoic acid at a yield of 84%.

### Example 10

### Preparation of benzoic acid:

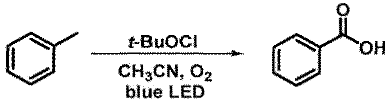

Example 10 differed from Example 1 in that tert-butyl hypochlorite was used as catalyst as follows.

Toluene (92 mg, 1 mmol), tert-butyl hypochlorite (t-BuOCl, 200 µL, 1 M), and acetonitrile (2 mL) were added in turn into a 25 mL quartz tube for mixing to obtain a mixture solution. The resulting mixture solution was irradiated with a blue LED lamp (40 W) with a wavelength of 380 nm to 550 nm in oxygen atmosphere (1 atm) for reacting for 12 h at room temperature. The solvent was removed under reduced pressure to obtain 0.112 g of the corresponding benzoic acid at a yield of 92%.

### Example 11

### Preparation of 4-phenylbenzoic acid:

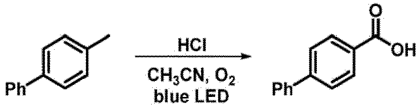

4-phenyltoluene (168mg, 1 mmol), hydrochloric acid (200 µL, 1 M), and acetonitrile (2 mL) were added in turn into a 25 mL quartz tube for mixing to obtain a mixture solution. The resulting mixture solution was irradiated with a blue LED lamp (40 W) with a wavelength of 380 nm to 550 nm in oxygen atmosphere (1 atm) for reacting for 12 h at room temperature. After the reaction was completed, the solvent was removed under reduced pressure to obtain 170 mg of the corresponding 4-phenylbenzoic acid at a yield of 86%.

The 1HNMR spectrum of the product was:
¹H NMR (400 MHz, DMSO) *δ* 13.04 (s, 1H), 8.05 (d, *J* = 8.4 Hz, 2H), 7.81 (d, *J* = 8.4 Hz, 2H), 7.74 (d, *J* = 7.2 Hz, 2H), 7.51 (t, *J* = 7.6 Hz, 2H), 7.44 (t, J= 7.2 Hz, 1H). ¹³C NMR (100 MHz, DMSO) *δ* 167.3, 144.4, 139.2, 130.1, 129.7, 129.2, 128.4, 127.1, 126.9.

The reaction product was demonstrated to be the target product 4-phenylbenzoic acid.

### Example 12

### Preparation of 1-indanone:

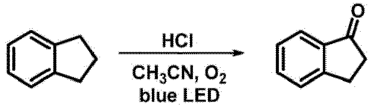

Indane (168 mg, 1 mmol), hydrochloric acid (200 µL, 1 M), and acetonitrile (2 mL) were added in turn into a 25 mL quartz tube for mixing to obtain a mixture solution. The resulting mixture solution was irradiated with a blue LED lamp (40 W) with a wavelength of 380 nm to 550 nm in oxygen atmosphere (1 atm) for reacting for 12 h at room temperature. After the reaction was completed, the solvent was removed under reduced pressure to obtain 103 mg of the corresponding 1-indanone at a yield of 78%.

The 1HNMR spectrum of the product was:
¹H NMR (400 MHz, CDCl₃) *δ* 7.77 (d, *J* = 7.6 Hz, 1H), 7.59 (t, *J* = 7.6 Hz, 1H), 7.49 (d, *J* = 7.6 Hz, 1H), 7.38 (t, *J* = 7.2 Hz, 1H), 3.17 - 3.14 (m, 2H), 2.72 - 2.69 (m, 2H). ¹³C NMR (100 MHz, CDCl₃) *δ* 207.1, 155.1, 137.1, 134.6, 127.3, 126.7, 123.7, 36.2, 25.8.

The reaction product was demonstrated to be the target product 1-indanone.

### Example 13

### Preparation of benzophenone:

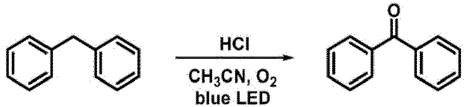

Diphenylmethane (168 mg, 1 mmol), hydrochloric acid (200 µL, 1 M), and acetonitrile (2 mL) were added in turn into a 25 mL quartz tube for mixing to obtain a mixture solution. The resulting mixture solution was irradiated with a blue LED lamp (40 W) with a wavelength of 380 nm to 550 nm in oxygen atmosphere (1 atm) for reacting for 24 h at room temperature. After the reaction was completed, the solvent was removed under reduced pressure to obtain 162 mg of the corresponding benzophenone at a yield of 89%.

The 1HNMR spectrum of the product was:
¹H NMR (400 MHz, CDCl₃) *δ* 7.81 (dd, *J* = 8.4, 1.3 Hz, 4H), 7.62-7.57 (m, 2H), 7.49 (t, *J* = 7.6 Hz, 4H). ¹³C NMR (100 MHz, CDCl₃) *δ* 132.4, 130.1, 128.3.

The reaction product was demonstrated to be the target product benzophenone.

### Example 14

### Oxidation of Celecoxib:

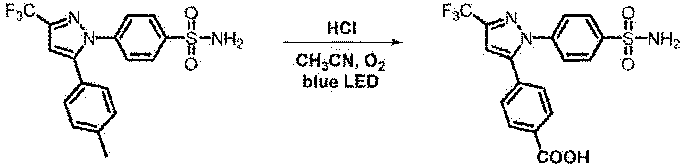

Celecoxib (381 mg, 1 mmol), hydrochloric acid (200 µL, 1 M), and acetonitrile (2 mL) were added in turn into a 25 mL quartz tube for mixing to obtain a mixture solution. The resulting mixture solution was irradiated with a blue LED lamp (40 W) with a wavelength of 380 nm to 550 nm in oxygen atmosphere (1 atm) for reacting for 24 h at room temperature. After the reaction was completed, the reaction solution was rapidly separated to obtain 262 mg of the corresponding oxidation product at a yield of 64%.

The 1HNMR spectrum of the product was:
¹H NMR (400 MHz, DMSO-d6) *δ* 12, 11 (brs, 1H), 7.94 (d, J= 8.4 Hz, 2H), 7.88 (d, *J* = 8.8 Hz, 2H), 7.55 (d, *J* = 8.8 Hz, 2H), 7.52 (s, 2H), 7.44 (d, *J* = 8.4 Hz, 2H), 7.35 (s, 1H). ¹³C NMR (100 MHz, DMSO-d6) *δ* 167.0, 144.6, 144.5, 142.5 (q, *J* = 10.0 Hz), 141.2, 132.6, 131.7, 130.0, 129.5, 127.3, 126.4, 120.3 (q, *J* = 247.0 Hz), 107.4. ¹⁹F NMR (377 MHz, DMSO-d6) *δ* -60.87.

The reaction product was demonstrated to be the target product.

### Example 15

### Oxidation of methane:

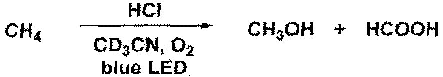

Hydrochloric acid (200 µL, 1 M) and deuterated acetonitrile (CD₃CN, 2 mL) were added in turn into a photoreactor, which was then tightened and filled with 20 atm of oxygen and 40 atm of methane. The resulting mixture solution was irradiated with a blue LED lamp (40 W) with a wavelength of 380 nm to 550 nm for reacting for 24 h at room temperature. After the reaction was completed, the yields for methanol and formic acid were determined to be 3.3% and16.5%, respectively, by NMR. The 1HNMR spectrum of the reaction product was as shown in FIG. 2.

The 1HNMR spectrum of formic acid was: ¹H NMR (400 MHz, CD3CN) δ 8.08 (s, 1H).

The 1HNMR spectrum of methanol was: ¹H NMR (400 MHz, CD3CN) δ 3.31 (s, 0.6 H).

### Example 16

### Oxidation of methane:

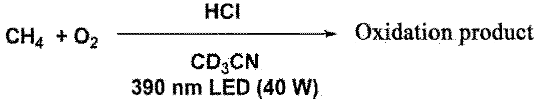

A stirrer was put into a 25 mL sealed tube, and hydrochloric acid (5.0 µL, 12.0 M) and deuterated acetonitrile (750.0 µL) were added in turn. The sealed tube was subjected to vacuum degassing under freezing conditions (-20°C) and filled with a gas mixture of methane and oxygen (a total pressure of the gas mixture of 1 atm, a gas pressure ratio of CH₄: O₂ = 1:1). The sealed tube was then placed in front of a LED lamp (40 W) with a wavelength of 390 nm and the reaction was carried out for 24 h at room temperature. After the reaction was completed, the corresponding oxidation product and the yield thereof were obtained via NMR, as shown in Table 1.

**Table 1**

| Oxidation product | Formic acid | Peroxymethanol | Methanol | Total Yield |
|---|---|---|---|---|
| Yield | 22.7% | 0.01% | 0.01% | 22.72% |

### Example 17

### Oxidation of ethane:

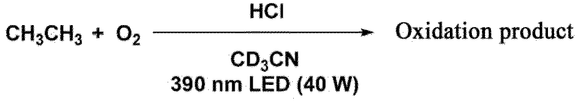

A stirrer was put into a 25 mL sealed tube, and hydrochloric acid (5.0 µL, 0.1 M) and deuterated acetonitrile (750.0 µL) were added in turn. The sealed tube was subjected to vacuum degassing under freezing conditions (-20°C) and filled with a gas mixture of ethane and oxygen (a total pressure of the gas mixture of 1 atm, a gas pressure ratio of ethane: O₂=1:1). The sealed tube was then placed in front of a LED lamp (40 W) with a wavelength of 390 nm and the reaction was carried out for 24 h at room temperature. After the reaction was completed, the corresponding oxidation product and the yield thereof were obtained via NMR, as shown in Table 2.

**Table 2**

| Oxidati on product | Formic acid | Acetic acid | Acetal dehyde | Peroxyeth anol | Ethano 1 | Formalde hyde | Peroxymeth anol | Metha nol | Total Yield |
|---|---|---|---|---|---|---|---|---|---|
| Yield | 7.92% | 8.14% | 1.53% | 1.50% | 2.23% | 0.03% | 0.43% | 1.33% | 23.11 % |

### Example 18

### Oxidation of propane:

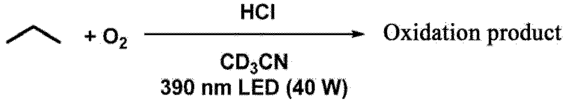

A stirrer was put into a 25 mL sealed tube, and hydrochloric acid (5.0 µL, 0.1 M) and deuterated acetonitrile (750.0 µL) were added in turn. The sealed tube was subjected to vacuum degassing under freezing conditions (-20°C) and filled with a gas mixture of propane and oxygen (a total pressure of the gas mixture of 1 atm, a gas pressure ratio of propane: O₂=1:1). The sealed tube was then placed in front of a LED lamp (40 W) with a wavelength of 390 nm and the reaction was carried out for 24 h at room temperature. After the reaction was completed, the corresponding oxidation product and the yield thereof were obtained via NMR, as shown in Table 3.

**Table 3**

| Oxidati on produc t | Aceto ne | Propio nic acid | Acet ic acid | Form ic acid | Propionalde hyde | n-Prop yl alcoh ol | Isopro pyl alcoho 1 | Acetaldeh yde | Ethan ol | Metha nol | Total Yield |
|---|---|---|---|---|---|---|---|---|---|---|---|
| Yield | 13.57 % | 5.77% | 4.75 % | 8.78 % | 0.26% | 5.57 % | 3.93% | 0.39% | 3.41 % | 2.43% | 48.86 % |

### Example 19

### Oxidation of butane:

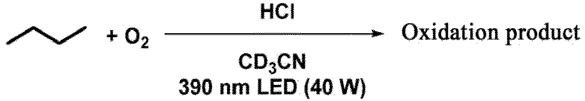

A stirrer was put into a 25 mL sealed tube, and hydrochloric acid (5.0 µL, 0.1 M) and deuterated acetonitrile (750.0 µL) were added in turn. The sealed tube was subjected to vacuum degassing under freezing conditions (-20°C) and filled with a gas mixture of butane and oxygen (a total pressure of the gas mixture of 1 atm, a gas pressure ratio of butane: O₂=1:1). The sealed tube was then placed in front of a LED lamp (40 W) with a wavelength of 390 nm and the reaction was carried out for 24 h at room temperature. After the reaction was completed, the corresponding oxidation product and the yield thereof were obtained via NMR, as shown in Table 4.

**Table 4**

| Oxidation product | 2-Butanone | Butyric acid | Acetic acid | Acetaldehyde | Formic acid | Ethanol | Methanol | Total Yield |
|---|---|---|---|---|---|---|---|---|
| Yield | 9.48% | 3.52% | 9.50% | 16.2% | 12.2% | 3.60% | 4.91% | 59.41% |

### Example 20

### Oxidation of gas mixture

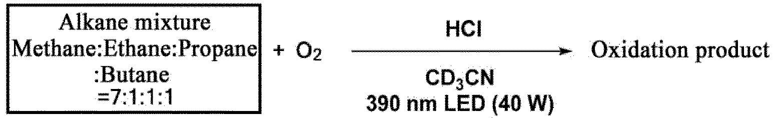

A stirrer was put into a 25 mL sealed tube, and hydrochloric acid (5.0 µL, 0.1 M) and deuterated acetonitrile (750.0 µL) were added in turn. The sealed tube was subjected to vacuum degassing under freezing conditions (-20°C) and filled with a gas mixture of mixed alkanes gas and oxygen (a total pressure of the gas mixture of 1 atm, a gas pressure ratio of methane:ethane:propane:butane: O₂=7:1:1:1:10), and the ratio of the mixed alkanes gas simulated the ratio of each alkane in natural gas. The sealed tube was then placed in front of a LED lamp (40 W) with a wavelength of 390 nm and the reaction was carried out for 24 h at room temperature. After the reaction was completed, the corresponding oxidation product and the yield thereof were obtained via NMR, as shown in Table 5.

**Table 5**

| Oxidation product | Formic acid | Acetic acid | Acetone | 2-Butanone | Propionic acid | Peroxymethanol | Methanol | Total Yield |
|---|---|---|---|---|---|---|---|---|
| Yield | 8.55% | 6.15% | 2.20% | 1.44% | 1.52% | 0.21% | 0.43% | 20.5% |

### Example 21

### Oxygen of petroleum ether:

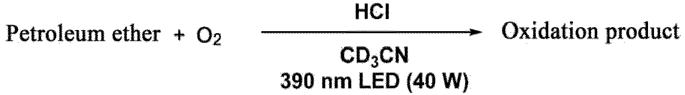

A stirrer was put into a 25 mL sealed tube, and hydrochloric acid (5.0 µL, 0.1 M), petroleum ether (22.6 mg), and deuterated acetonitrile (750.0 µL) were added in turn. The sealed tube was subjected to vacuum degassing under freezing conditions (-20°C) and filled with oxygen (1 atm). The sealed tube was then placed in front of a LED lamp (40 W) with a wavelength of 390 nm and the reaction was carried out for 24 h at room temperature. After the reaction was completed, the corresponding oxidation product and the yield thereof were obtained via NMR, as shown in Table 6.

**Table 6**

| Oxidation product | Acetone | Formic acid | Acetic acid | Acetaldehyde | Ethanol | Methanol | Total Yield |
|---|---|---|---|---|---|---|---|
| Yield | 12.30% | 9.73% | 8.50% | 3.38% | 4.80% | 3.95% | 42.66% |

### Example 22

### Oxidation of n-hexane:

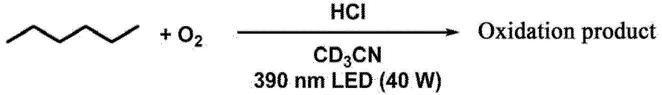

A stirrer was put into a 25 mL sealed tube, and hydrochloric acid (5.0 µL, 0.1 M), n-hexane (17.2 mg), and deuterated acetonitrile (750.0 µL) were added in turn. The sealed tube was subjected to vacuum degassing under freezing conditions (-20°C) and filled with oxygen (1 atm). The sealed tube was then placed in front of a LED lamp (40 W) with a wavelength of 390 nm and the reaction was carried out for 24 h at room temperature. After the reaction was completed, the corresponding oxidation product and the yield thereof were obtained via NMR, as shown in Table 7.

**Table 7**

| Oxidation product | Acetone | Formic acid | Acetic acid | Acetaldehyde | Methanol | Hexan-2-one | Total Yield |
|---|---|---|---|---|---|---|---|
| Yield | 3.50% | 5.46% | 7.00% | 3.78% | 0.98% | 8.82% | 29.54% |

### Example 23

### Oxidation of cyclohexane:

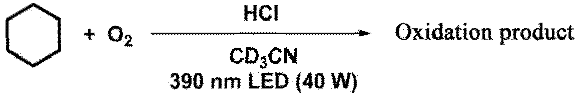

A stirrer was put into a 25 mL sealed tube, and hydrochloric acid (5.0 µL, 0.1 M), cyclohexane (16.8 mg), and deuterated acetonitrile (750.0 µL) were added in turn. The sealed tube was subjected to vacuum degassing under freezing conditions (-20°C) and filled with oxygen (1 atm). The sealed tube was then placed in front of a LED lamp (40 W) with a wavelength of 390 nm and the reaction was carried out for 24 h at room temperature. After the reaction was completed, the corresponding oxidation product and the yield thereof were obtained via NMR, as shown in Table 8.

**Table 8**

| Oxidation product | Formic acid | Acetaldehyde | Cyclohexanone | Cyclohexanol | Total Yield |
|---|---|---|---|---|---|
| Yield | 3.73% | 1.70% | 12.65% | 11.2% | 29.28% |

### Example 24

### Oxidation of isooctane:

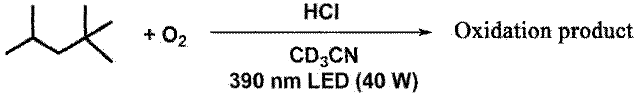

A stirrer was put into a 25 mL sealed tube, and hydrochloric acid (5.0 µL, 0.1 M), isooctane (22.8 mg), and deuterated acetonitrile (750.0 µL) were added in turn. The sealed tube was subjected to vacuum degassing under freezing conditions (-20°C) and filled with oxygen (1 atm). The sealed tube was then placed in front of a LED lamp (40 W) with a wavelength of 390 nm and the reaction was carried out for 24 h at room temperature. After the reaction was completed, the corresponding oxidation product and the yield thereof were obtained via NMR, as shown in Table 9.

**Table 9**

| Oxidation product | Acetone | Formic acid | Methanol | Pivalaldehyde | Total Yield |
|---|---|---|---|---|---|
| Yield | 0.79% | 4.75% | 1.59% | 2.38% | 9.51% |

### Example 25

### Oxidation of n-heptane:

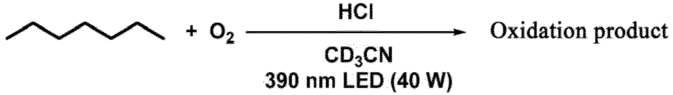

A stirrer was put into a 25 mL sealed tube, and hydrochloric acid (5.0 µL, 0.1 M), n-heptane (20.0 mg), and deuterated acetonitrile (750.0 µL) were added in turn. The sealed tube was subjected to vacuum degassing under freezing conditions (-20°C) and filled with oxygen (1 atm). The sealed tube was then placed in front of a LED lamp (40 W) with a wavelength of 390 nm and the reaction was carried out for 24 h at room temperature. After the reaction was completed, the corresponding oxidation product and the yield thereof were obtained via NMR, as shown in Table 10.

**Table 10**

| Oxidation product | Acetone | Formic acid | Methanol | Acetic acid | Acetaldehyde | Ethanol | 2-Heptanone | Total Yield |
|---|---|---|---|---|---|---|---|---|
| Yield | 2.4% | 6.7% | 1.3% | 8.0% | 3.8% | 3.3% | 6.4% | 31.9% |

### Example 26

### Oxidation of n-octane:

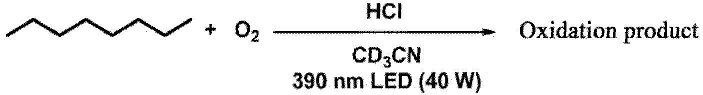

A stirrer was put into a 25 mL sealed tube, and hydrochloric acid (5.0 µL, 0.1 M), n-octane (22.8 mg), and deuterated acetonitrile (750.0 µL) were added in turn. The sealed tube was subjected to vacuum degassing under freezing conditions (-20°C) and filled with oxygen (1 atm). The sealed tube was then placed in front of a LED lamp (40 W) with a wavelength of 390 nm and the reaction was carried out for 24 h at room temperature. After the reaction was completed, the corresponding oxidation product and the yield thereof were obtained via NMR, as shown in Table 11.

**Table 11**

| Oxidation product | Acetone | Formic acid | Methanol | Acetic acid | Acetaldehyde | 2-Octanone | Total Yield |
|---|---|---|---|---|---|---|---|
| Yield | 1.3% | 2.3% | 0.5% | 3.5% | 5.0% | 4.1% | 16.7% |

### Example 27

### Oxidation of cyclohexane:

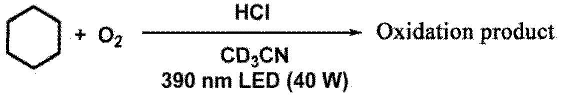

A stirrer was put into a 25 mL sealed tube, and hydrochloric acid (5.0 µL, 0.1 M), cyclohexane (16.8 mg), and deuterated acetonitrile (750.0 µL) were added in turn. The sealed tube was subjected to vacuum degassing under freezing conditions (-20°C) and filled with air (1 atm). The sealed tube was then placed in front of a LED lamp (40 W) with a wavelength of 390 nm and the reaction was carried out for 24 h at room temperature. After the reaction was completed, the corresponding oxidation product and the yield thereof were obtained via NMR, as shown in Table 12.

**Table 12**

| Oxidation product | Formic acid | Acetaldehyde | Cyclohexanone | Cyclohexanol | Total Yield |
|---|---|---|---|---|---|
| Yield | 2.67% | 1.22% | 10.56% | 9.04% | 23.49% |

According to the method for light-promoted oxidation of a compound containing a saturated carbon-hydrogen bond provided by the Examples of the present application, the compound containing a saturated carbon-hydrogen bond undergoes an oxidation reaction with oxygen in the presence of catalyst under a condition of light irradiation to synthesize a corresponding oxidation product, such as at least one of alcohol, peroxyalcohol, aldehyde, ketone, carboxylic acid, or peroxyacid. The method is a light-promoted direct oxidation method of a compound containing a saturated carbon-hydrogen bond, which only needs to be carried out at a low temperature (20°C to100°C) and has the advantages of good compatibility of functional groups, short reaction time, high reaction efficiency, low reaction cost, high added value, simple operation, and good safety. Moreover, It uses only common acids instead of transition metals as catalyst, and requires only light to promote the reaction to occur. Thus this method is milder, greener, and more environmentally friendly oxidation method.

The above embodiments are preferred Examples of the present application and are not intended to limit the scope of protection of the present application. Any modifications, equivalent substitutions, improvements and the like made within the spirit and principles of the present application should be included in the scope of protection of the present application.

## Claims

1. A method for light-promoted oxidation of a compound containing a saturated carbon-hydrogen bond, comprising: mixing the compound containing the saturated carbon-hydrogen bond with a catalyst, and subjecting the compound containing the saturated carbon-hydrogen bond to an oxidation reaction in an oxygen or air atmosphere at a temperature of 20°C to 100°C under light irradiation to generate an oxidation product.

2. The method for light-promoted oxidation of the compound containing the saturated carbon-hydrogen bond according to claim 1, wherein the catalyst is any one of tert-butyl hypochlorite, hydrochloric acid or hydrobromic acid; or
the catalyst is a mixture capable of in-situ generating hydrochloric acid or hydrobromic acid, wherein the mixture is a mixture of chloride and acetic acid or of bromide and acetic acid.

3. The method for light-promoted oxidation of the compound containing the saturated carbon-hydrogen bond according to claim 1, wherein the compound containing the saturated carbon-hydrogen bond is C1-C8 alkane.

4. The method for light-promoted oxidation of the compound containing the saturated carbon-hydrogen bond according to claim 1, wherein the compound containing the saturated carbon-hydrogen bond is C1-C8 alkane substituted with phenyl, biphenyl or substituted phenyl, wherein a substituent of the substituted phenyl is any one selected from the group consisting of halogen, nitro, cyano, hydroxyl, amino, carboxyl, C2-C8 carboxylic ester group and

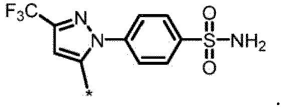

5. The method for light-promoted oxidation of the compound containing the saturated carbon-hydrogen bond according to claim 1, wherein the catalyst is an oxyacid, wherein the oxyacid is any one of p-toluenesulfonic acid, sulfuric acid or nitric acid.

6. The method for light-promoted oxidation of the compound containing the saturated carbon-hydrogen bond according to claim 1, wherein the light has a wavelength of 300 nm to 800 nm.

7. The method for light-promoted oxidation of the compound containing the saturated carbon-hydrogen bond according to claim 1, wherein the oxidation product is at least one of alcohol, peroxyalcohol, aldehyde, ketone, carboxylic acid or peroxyacid.

8. The method for light-promoted oxidation of the compound containing the saturated carbon-hydrogen bond according to claim 1, wherein the oxygen or air atmosphere in a reaction system has a pressure of 1 atm to 100 atm.

9. The method for light-promoted oxidation of the compound containing the saturated carbon-hydrogen bond according to claim 1, wherein a molar ratio of the compound containing the saturated carbon-hydrogen bond to the catalyst is 1:0.0001 to 1:1.

10. The method for light-promoted oxidation of the compound containing the saturated carbon-hydrogen bond according to claim 1, wherein a solvent for the oxidation reaction is any one of acetonitrile, tetrahydrofuran, dichloromethane, dichloroethane, trichloromethane, dimethyl sulfoxide, dibromoethane or N,N-dimethylformamide.
